# EUROPEAN PATENT APPLICATION

(11) **EP 2 457 583 A1**
(43) Date of publication of application: **30.05.2012**
(21) Application number: 10192693.9
(22) Date of filing: 26.11.2010
(51) Int. Cl.: A61K 39/12

(54) **Nucleic acids encoding PRRSV GP5-ecto and M protein**

(71) Applicant: CONSEJO SUPERIOR DE INVESTIGACIONES CIENTIFICAS, 28006 Madrid (ES); Fort Dodge Veterinaria, S.A., 17813 Vall de Bianya Gerona (ES)
(72) Inventor: Enjuanes Sanchez, Luis, 28109 Madrid (ES); Plana Duran, Joan, 17811 Santa Pau, Girona (ES); Zuniga Lucas, Sonia, 28760 Madrid (ES); Becares Palacios, Martina, 49159 Villaralbo Zamora (ES)
(74) Representative: UEXKÜLL & STOLBERG

(57) **Abstract**

The present invention relates to nucleic acids comprising:
(a) a sequence encoding a polypeptide comprising the amino acid sequence of SEQ ID NO:1 (sequence of the GP5 ecto-domain of the European strain of PRRSV) or a sequence having at least 85% identity to SEQ ID NO:1, which sequence includes at least one amino acid capable of forming a disulfide bridge; and
(b) a sequence encoding a polypeptide comprising the amino acid sequence SEQ ID NO:2 (sequence of the GP5 M protein of the European strain of PRRSV) or a sequence having at least 85% identity to SEQ ID NO: 2;
(c) wherein the nucleic acid does not comprise a sequence encoding a polypeptide comprising the amino acid sequence of SEQ ID NO:3 (sequence of the GP5 protein except the ecto-domain of the European strain of PRRSV) or a sequence having at least 80% identity to SEQ ID NO:3.

The present invention further provides respective nucleic acids from other strains of PRRSV and their use as a vaccine for the treatment of animals.

SEQUENCE LISTING

<110>  Consejo Superior de Investigaciones Cientificas

<120>  NUCLEIC ACIDS ENCODING PRRSV GP5-ECTO AND M PROTEIN

<130>  P84080

<160>  11

<170>  PatentIn version 3.3

<210>  1
<211>  68
<212>  PRT
<213>  Porcine reproductive and respiratory syndrome virus

<220>
<221>  misc_feature
<223>  Gp5 ecto domain sequence of PRRSV strain Olot91.

<400>  1

Met Arg Cys Ser His Lys Leu Gly Arg Phe Leu Thr Pro His Ser Cys
1               5                   10                  15

Phe Trp Trp Leu Phe Leu Leu Cys Thr Gly Leu Ser Trp Ser Phe Val
            20                  25                  30

Ala Gly Ser Gly Ser Ser Ser Thr Tyr Gln Tyr Ile Tyr Asn Leu Thr
        35                  40                  45

Ile Cys Glu Leu Asn Gly Thr Asp Trp Leu Ser Asn His Phe Asp Trp
    50                  55                  60

Ala Val Glu Thr
65

<210> 2
<211> 173
<212> PRT
<213> porcine reproductive and respiratory syndrome virus

<220>
<221> misc_feature
<223> M protein sequence of PRRSV strain Olot91.

<400> 2

Met Gly Ser Leu Asp Asp Phe Cys Asn Asp Ser Thr Ala Ala Gln Lys
1               5                   10                  15

Leu Val Leu Ala Phe Ser Ile Thr Tyr Thr Pro Ile Met Ile Tyr Ala
            20                  25                  30

Leu Lys Val Ser Arg Gly Arg Leu Leu Gly Leu Leu His Ile Leu Ile
        35                  40                  45

Phe Leu Asn Cys Ser Phe Thr Phe Gly Tyr Met Thr Tyr Val Arg Phe
    50                  55                  60

Gln Ser Thr Asn Arg Val Ala Leu Thr Leu Gly Ala Val Val Ala Leu
65                  70                  75                  80

Leu Trp Gly Val Tyr Ser Phe Thr Glu Ser Trp Lys Phe Val Thr Ser
                85                  90                  95

Arg Cys Arg Leu Cys Cys Leu Gly Arg Arg Tyr Ile Leu Ala Pro Ala
            100                 105                 110

His His Val Glu Ser Ala Ala Gly Leu His Ser Ile Pro Ala Ser Gly
        115                 120                 125

Asn Arg Ala Tyr Ala Val Arg Lys Pro Gly Leu Thr Ser Val Asn Gly
    130                 135                 140

Thr Leu Val Pro Gly Leu Arg Ser Leu Val Leu Gly Gly Lys Arg Ala
145                 150                 155                 160

Val Lys Arg Gly Val Val Asn Leu Val Lys Tyr Gly Arg
                165                 170

<210>  3
<211>  133
<212>  PRT
<213>  porcine reproductive and respiratory syndrome virus

<220>
<221>  misc_feature
<223>  Gp5 protein sequence lacking the ecto domain sequence of
PRRSV strain Olot91.

<400>  3

Phe Val Leu Tyr Pro Val Ala Thr His Ile Leu Ser Leu Gly Phe Leu

1               5                   10                  15

Thr Thr Ser His Phe Phe Asp Ala Leu Gly Leu Gly Ala Val Ser Thr
            20                  25                  30

Thr Gly Phe Val Gly Gly Arg Tyr Val Leu Ser Ser Val Tyr Gly Ala
        35                  40                  45

Cys Ala Phe Ala Ala Leu Val Cys Phe Val Ile Arg Ala Val Lys Asn
    50                  55                  60

Cys Met Ala Cys Arg Tyr Ala His Thr Arg Phe Thr Asn Phe Ile Val
65                  70                  75                  80

Asp Asp Arg Gly Arg Ile His Arg Trp Lys Ser Pro Ile Val Val Glu
                85                  90                  95

Lys Leu Gly Lys Ala Glu Val Gly Gly Asp Leu Val Thr Ile Lys His
            100                 105                 110

Val Val Leu Glu Gly Val Lys Ala Gln Pro Leu Thr Arg Thr Ser Ala
        115                 120                 125

Glu Gln Trp Glu Ala
    130

<210>  4
<211>  66
<212>  PRT

<213>  Porcine reproductive and respiratory syndrome virus

<220>
<221>  misc_feature
<223>  Gp5 ecto domain sequence of PRRSV strain VR2332.

<400>  4

Met Leu Glu Lys Cys Leu Thr Ala Gly Cys Cys Ser Arg Leu Leu Ser
1               5                   10                  15

Leu Trp Cys Ile Val Pro Phe Cys Phe Ala Val Leu Ala Asn Ala Ser
            20                  25                  30

Asn Asp Ser Ser Ser His Leu Gln Leu Ile Tyr Asn Leu Thr Leu Cys
        35                  40                  45

Glu Leu Asn Gly Thr Asp Trp Leu Ala Asn Lys Phe Asp Trp Ala Val
    50                  55                  60

Glu Ser
65

<210>  5
<211>  174
<212>  PRT
<213>  Porcine reproductive and respiratory syndrome virus

<220>
<221>  misc_feature
<223>  M protein sequence of PRRSV strain VR2332.

<400>  5

Met Gly Ser Ser Leu Asp Asp Phe Cys His Asp Ser Thr Ala Pro Gln
1               5                   10                  15

Lys Val Leu Leu Ala Phe Ser Ile Thr Tyr Thr Pro Val Met Ile Tyr
            20                  25                  30

Ala Leu Lys Val Ser Arg Gly Arg Leu Leu Gly Leu Leu His Leu Leu
        35                  40                  45

Ile Phe Leu Asn Cys Ala Phe Thr Phe Gly Tyr Met Thr Phe Ala His
    50                  55                  60

Phe Gln Ser Thr Asn Lys Val Ala Leu Thr Met Gly Ala Val Val Ala
65                  70                  75                  80

Leu Leu Trp Gly Val Tyr Ser Ala Ile Glu Thr Trp Lys Phe Ile Thr
                85                  90                  95

Ser Arg Cys Arg Leu Cys Leu Leu Gly Arg Lys Tyr Ile Leu Ala Pro
            100                 105                 110

Ala His His Val Glu Ser Ala Ala Arg Phe His Pro Ile Ala Ala Asn
        115                 120                 125

Asp Asn His Ala Phe Val Val Arg Arg Pro Gly Ser Thr Thr Val Asn
    130                 135                 140

Gly Thr Leu Val Pro Gly Leu Lys Ser Leu Val Leu Gly Gly Arg Lys
145                 150                 155                 160

Ala Val Lys Gln Gly Val Val Asn Leu Val Lys Tyr Ala Lys
                165                 170

<210>  6
<211>  134
<212>  PRT
<213>  Porcine reproductive and respiratory syndrome virus

<220>
<221>  misc_feature
<223>   Gp5 protein sequence lacking the ecto domain sequence of
PRRSV strain VR2332.

<400>  6

Phe Val Ile Phe Pro Val Leu Thr His Ile Val Ser Tyr Gly Ala Leu
1               5                   10                  15

Thr Thr Ser His Phe Leu Asp Thr Val Ala Leu Val Thr Val Ser Thr
            20                  25                  30

Ala Gly Phe Val His Gly Arg Tyr Val Leu Ser Ser Ile Tyr Ala Val
        35                  40                  45

Cys Ala Leu Ala Ala Leu Thr Cys Phe Val Ile Arg Phe Ala Lys Asn
    50                  55                  60

Cys Met Ser Trp Arg Tyr Ala Cys Thr Arg Tyr Thr Asn Phe Leu Leu
65                  70                  75                  80

Asp Thr Lys Gly Arg Leu Tyr Arg Trp Arg Ser Pro Val Ile Ile Glu
                85                  90                  95

Lys Arg Gly Lys Val Glu Val Glu Gly His Leu Ile Asp Leu Lys Arg
            100                 105                 110

Val Val Leu Asp Gly Ser Val Ala Thr Pro Ile Thr Arg Val Ser Ala
        115                 120                 125

Glu Gln Trp Gly Arg Pro
    130

<210>  7
<211>  66
<212>  PRT
<213>  Porcine reproductive and respiratory syndrome virus

<220>
<221>  misc_feature
<223>  Gp5 ecto domain sequence of  PRRSV strain HB-2(sh).

<400>  7

Met Leu Gly Lys Cys Leu Thr Ala Gly Cys Tyr Ser Gln Leu Leu Ser
1               5                   10                  15

Leu Trp Cys Ile Val Pro Phe Cys Phe Ala Val Leu Val Asn Ala Ser
            20                  25                  30

Ser Asn Ser Ser Pro His Phe Gln Leu Ile Tyr Asn Leu Thr Leu Cys
        35                  40                  45

Glu Leu Asn Gly Thr Asp Trp Leu Ala Glu Arg Phe Asp Trp Ala Val
    50                  55                  60

Glu Thr
65

<210>  8
<211>  174
<212>  PRT
<213>  Porcine reproductive and respiratory syndrome virus

<220>
<221>  misc_feature
<223>  M protein sequence of  PRRSV strain HB-2(sh).

<400>  8

Met Gly Ser Ser Leu Asp Asp Phe Cys His Asp Ser Thr Ala Pro Gln
1               5                   10                  15

Lys Val Leu Leu Ala Phe Ser Ile Thr Tyr Thr Pro Val Met Ile Tyr
            20                  25                  30

Ala Leu Lys Val Ser Arg Gly Arg Leu Leu Gly Leu Leu His Leu Leu
        35                  40                  45

Ile Phe Leu Asn Cys Ala Phe Thr Phe Gly Tyr Met Thr Phe Val His
    50                  55                  60

Phe Gln Ser Thr Asn Arg Val Ala Leu Thr Met Gly Ala Val Val Ala
65                  70                  75                  80

Leu Leu Trp Gly Val Tyr Ser Ala Ile Glu Thr Trp Lys Phe Ile Thr
                85                  90                  95

Ser Arg Cys Arg Leu Cys Leu Leu Gly Arg Lys Tyr Ile Leu Ala Pro
            100                 105                 110

Ala His His Val Glu Ser Ala Ala Gly Phe His Pro Ile Ala Ala Asn
        115                 120                 125

Asp Asn His Ala Phe Val Val Arg Arg Pro Gly Ser Thr Thr Val Asn
    130                 135                 140

Gly Ile Leu Val Pro Gly Leu Lys Ser Leu Val Leu Gly Gly Arg Lys
145                 150                 155                 160

Ala Val Lys Gln Gly Val Val Asn Leu Val Lys Tyr Ala Lys
                165                 170

<210>  9
<211>  134
<212>  PRT
<213>  Porcine reproductive and respiratory syndrome virus

<220>
<221> misc_feature
<223> Gp5 protein sequence lacking the ecto domain sequence of
PRRSV strain HB-2(sh).

<400> 9

Phe Val Ile Phe Pro Val Leu Thr His Ile Val Ser Tyr Gly Ala Leu
1               5                   10                  15

Thr Thr Ser His Phe Leu Asp Thr Val Gly Leu Ile Thr Val Ser Thr
            20                  25                  30

Ala Gly Phe Tyr His Arg Arg Tyr Val Leu Ser Ser Ile Tyr Ala Val
        35                  40                  45

Cys Ala Leu Ala Ala Leu Ile Cys Phe Val Ile Arg Leu Ala Lys Asn
    50                  55                  60

Cys Met Ser Trp Arg Tyr Ser Cys Thr Arg Tyr Thr Asn Phe Leu Leu
65                  70                  75                  80

Asp Thr Lys Gly Arg Leu Tyr Arg Trp Arg Pro Pro Val Ile Val Glu
                85                  90                  95

Lys Gly Gly Lys Val Glu Val Glu Gly His Leu Ile Asp Leu Lys Arg
            100                 105                 110

Val Val Leu Asp Gly Ser Val Ala Thr Pro Leu Thr Arg Val Ser Ala
        115                 120                 125

Glu Gln Trp Gly Arg Leu
    130

<210>  10
<211>  5
<212>  PRT
<213>  Artificial

<220>
<223>  flexible linker sequence

<400>  10

Gly Gly Pro Gly Gly
1               5

<210>  11
<211>  9
<212>  PRT
<213>  Artificial

<220>
<223>  Epitope tag

<400>  11

Tyr Pro Tyr Asp Val Pro Asp Tyr Ala
1               5

<210>  12
<211>  63
<212>  PRT
<213>  Porcine reproductive and respiratory syndrome virus

<220>

<221>  misc_feature

<223>  Gp5 ecto domain sequence of European PRRSV strain Olot91 with
       N46S mutation and deletion of amino acids 30-34

<400>  12

Met Arg Cys Ser His Lys Leu Gly Arg Phe Leu Thr Pro His Ser Cys
1               5                   10                  15

Phe Trp Trp Leu Phe Leu Leu Cys Thr Gly Leu Ser Trp Ser Gly Ser
            20                  25                  30

Ser Ser Thr Tyr Gln Tyr Ile Tyr Ser Leu Thr Ile Cys Glu Leu Asn
        35                  40                  45

Gly Thr Asp Trp Leu Ser Asn His Phe Asp Trp Ala Val Glu Thr
    50                  55                  60

## Description

The present invention relates to nucleic acids comprising sequences encoding specific fragments of the GP5 protein and the M protein of PRRSV. The present invention further relates to recombinant RNA and polypeptides encoded by the nucleic acids of the present invention, to vectors, virus particles and host cells comprising the nucleic acids of the invention as well as their use for the preparation of pharmaceutical compositions and vaccines.

### TECHNICAL BACKGROUND

Porcine reproductive and respiratory syndrome virus (PRRSV) was first identified in 1991 as the causative agent of a new disease in pigs (Wensvoort et al., 1991). Since then, PRRSV has become one of the leading causes of economic losses in swine operations worldwide and it is currently accepted as the most important infectious disease of swine, causing reproductive failure in adult animals and severe pneumonia in neonatal pigs. PRRSV is a member of *Arteriviridae* family that belongs to *Nidovirales* order. Two different genotypes (American and European) are known and potentially a third genotype has recently been identified (Shi et al., 2010; Murtaugh et al., 1995). PRRSV is an enveloped virus with a single-stranded positive-sense RNA genome of 14.5 Kb. The 5' two-thirds of the genome encode the replicase polyproteins (pp1a and pp1ab). The rest of the genomic RNA encodes four minor membrane-associated proteins (E, Gp2, Gp3 and Gp4) and the three major structural proteins (Gp5, M and N) (Dokland, 2010).

Pigs are generally infected with PRRSV following exposure of the mucosal surface or the respiratory tract to the virus. A hallmark of the swine antibody response against PRRSV are the abundant non-neutralizing antibodies detected early in the infection, followed by a low neutralizing antibody titer that appears more than 3 weeks after infection. Experimental data showing the importance of neutralizing antibodies for protection against PRRSV infection have been collected in the last years (Lopez & Osorio, 2004).

The immune response to PRRSV is not fully understood but, in spite of this, some vaccines are being commercialized. Modified live vaccines, i.e. attenuated microorganisms with reduced virulence, protect against challenges with homologous isolates, but generally have a limited effect against challenges with heterologous viruses (Meng, 2000). Furthermore, although live vaccines provide partial protection against the clinical symptoms of disease they do not prevent subsequent infection (Osorio et al., 1998). As the attenuated vaccines induce an immune response resembling that induced by PRRSV natural infection, they do not induce high levels of neutralizing antibodies. Even more importantly, live vaccines can revert to virulence (Botner et al., 1997, Nielsen et al., 2001).

One frequently used alternative to attenuated live vaccines are inactivated viruses, which have been treated with chemicals or heat. However, inactivated PRRSV vaccines have proven to be less effective in prevention of both infection and disease (Ostrowski et al, 2002).

The incomplete protection provided by vaccines based on attenuated or "killed" PRRSV has led to an increase in the use of potentially hazardous approaches to control the disease, including the use of live field virus to vaccinate pigs. Additionally, safer and more reliable vector-based vaccines have been developed and tested (Cruz et al., 2010). These vector-based vaccines could have the advantage to stimulate both humoral and cell immune responses against PRRSV and therefore elicit sufficiently strong immune responses. In line with this strategy, vectors derived from the genome of a coronavirus, the Transmissible Gastroenteritis Virus (TGEV), have been used, which express combinations of different PRRSV antigens (EP 1 792 996). For example, a recombinant TGEV vector expressing PRRSV membrane proteins GP5 (wild-type or modified) and M protein was tested as a vaccine and led to positive results, such as induction of a humoral immune response against PRRSV antigens, and protection in lungs (Cruz et al., 2010). The GP5 protein forms a heterodimer with the M protein in the viral membrane. This heterodimer is involved in receptor recognition and viral neutralization.

However, problems were encountered due to instability of the gene expressing the GP5 protein cloned in the recombinant TGEV (rTGEV) vector in the respective host cells. While the expression of the M protein from rTGEV was fully stable, tissue culture passages of rTGEV viruses led to a partial loss of GP5 protein expression. After 10 passages in cell culture the numbers of recombinant viruses which expressed GP5 were reduced by 80%. This result suggests that GP5 protein may contain toxic domains for a host cell which expresses recombinant GP5 protein.

The problem underlying the present invention thus resides in providing PRRSV sequences which will provide stabilized vectors and thus improved pharmaceutical compositions and vaccines for the treatment of PRRSV.

### SUMMARY OF THE INVENTION

The present invention provides a nucleic acid comprising:
(a) a sequence encoding a polypeptide comprising the amino acid sequence of SEQ ID NO:1 (amino acids 1 to 68 of GP5 of the European PRRSV strain) or a sequence having at least 85% or at least 90% identity to SEQ ID NO:1, which sequence includes at least one amino acid capable of forming a disulfide bridge; and
(b) a sequence encoding a polypeptide comprising the amino acid sequence SEQ ID NO:2 (the sequence of the M protein of the European PRRSV strain) or a sequence having at least 85% or at least 90% identity to SEQ ID NO:2;
(c) wherein the nucleic acid does not comprise a sequence encoding a polypeptide comprising the amino acid sequence of SEQ ID NO:3 (amino acids 69 to 201 of GP5 of the European PRRSV strain) or a sequence having at least 80% identity to SEQ ID NO:3.

The present invention also provides corresponding nucleic acids for the respective sequences of the North American and Chinese strain of PRRSV.

The nucleic acids of the invention may encode a fusion protein comprising the sequences of (a) and (b) or two separate polypeptides (a) and (b).

According to one embodiment, the nucleic acids of the present invention may comprise more than one copy of SEQ ID NO:1 or a sequence having at least 85% or at least 90% identity to SEQ ID NO:1. In other words, the invention encompasses nucleic acids encoding multimers of SEQ ID NO:1 and sequences encoding a polypeptide comprising the amino acid sequence SEQ ID NO:2 (European PRRSV strain).

The nucleic acids of the invention may encode further PRRSV proteins (such as GP2, GP3 or GP4) and/or a lysosomal targeting sequence of LIMP-II and/or genetic adjuvants (such as Interleukins).

In the nucleic acids of the present invention sequences (a) and (b) as defined above may be present under the control of the same expression regulatory sequence. Alternatively, sequences (a) and (b) as defined above may be present in the nucleic acids under the control of a different expression regulatory sequence.

The present invention further provides polypeptides encoded by these nucleic acids, vectors, virus particles and host cells comprising these nucleic acids as well as their use for the preparation of pharmaceutical preparations and vaccines.

### DETAILED DESCRIPTION OF THE INVENTION

According to one embodiment, the present invention is thus directed to a nucleic acid which comprises
(a) a sequence encoding a polypeptide comprising the amino acid sequence of SEQ ID NO:1 (amino acids 1 to 68 of GP5 of the European PRRSV strain) or a sequence having at least 85% or at least 90% identity to SEQ ID NO:1, which sequence includes at least one amino acid capable of forming a disulfide bridge; and
(b) a sequence encoding a polypeptide comprising the amino acid sequence SEQ ID NO:2 (the sequence of the M protein of the European PRRSV strain) or a sequence having at least 85% or at least 90% identity to SEQ ID NO: 2;
(c) wherein the nucleic acid does not comprise a sequence encoding a polypeptide comprising the amino acid sequence of SEQ ID NO:3 (amino acids 69 to 201 of GP5 of the European PRRSV strain) or a sequence having at least 80% identity to SEQ ID NO:3.

The present invention is also directed to a nucleic acid which comprises:
(a) a sequence encoding a polypeptide comprising the amino acid sequence of SEQ ID NO:4 (amino acids 1 to 66 of GP5 of the North American PRRSV strain) or a sequence having at least 85% or at least 90% identity to SEQ ID NO:4, which sequence includes at least one amino acid capable of forming a disulfide bridge; and
(b) a sequence encoding a polypeptide comprising the amino acid sequence SEQ ID NO:5 (the sequence of the M protein of the North American PRRSV strain) or a sequence having at least 85% or at least 90% identity to SEQ ID NO:5;
(c) wherein the nucleic acid does not comprise a sequence encoding a polypeptide comprising the amino acid sequence of SEQ ID NO:6 (amino acids 67 to 200 of GP5 of the North American PRRSV strain) or a sequence having at least 80% identity to SEQ ID NO:6.

The present invention is also directed to a nucleic acid which comprises:
(a) a sequence encoding a polypeptide comprising the amino acid sequence of SEQ ID NO:7 (amino acids 1 to 66 of GP5 of the Chinese PRRSV strain) or a sequence having at least 85% or at least 90% identity to SEQ ID NO:7, which sequence includes at least one amino acid capable of forming a disulfide bridge; and
(b) a sequence encoding a polypeptide comprising the amino acid sequence SEQ ID NO:8 (the sequence of the M protein of the Chinese PRRSV strain) or a sequence having at least 85% or at least 90% identity to SEQ ID NO:8;
(c) wherein the nucleic acid does not comprise a sequence encoding a polypeptide comprising the amino acid sequence of SEQ ID NO:9 (amino acids 67 to 200 of GP5 of the Chinese PRRSV strain) or a sequence having at least 80% identity to SEQ ID NO:9.

Further representative strains of Chinese PRRSV are described in published Chinese application CN 200910091233.6 (CN 101633909A). The nucleotide and amino acid sequences can be aligned with other Chinese or North American strains and European strains, in a straightforward manner. In a further embodiment, the sequences of these further Chinese PRRSV strains can be used to generate nucleic acids of the present invention, as will be immediately appreciated by those skilled in the art.

For the purposes of the present invention, sequence identity is determined using the ClustalW computer program available from the European Bioinformatics Institute (EBI).

The present application for the first time shows that expression of the GP5 protein of PRRSV can be stabilized by expressing the GP5 ecto-domain without the further sequences of this polypeptide. In fact, expression of the GP5 ecto-domain was very similar to the expression previously obtained for the PRRSV M protein. Further, the expression levels remained stable for more than 10 virus passages in tissue culture. The present inventors have thus surprisingly found that the stability of the expression of the GP5 polypeptide can be significantly improved by expression of the ecto-domain of GP5 without the further amino acids of the natural GP5 polypeptide. Together with the M protein of PRRSV the nucleic acids of the present invention thus provide a stable construct that can advantageously be used for the preparation of vaccines.

While the present invention encompasses the use of sequences from different strains of PRRSV, the use of the sequences of the European strain of PRRSV (the sequence of the strain Olot91) is most preferred in all aspects of the present invention.

The GP5 ecto-domain contains the cysteine residue that forms disulfide bridges between GP5 and M proteins. The GP5 ecto-domain subdomains which are involved in raising an immune response are presumably the immunodominant epitope (IDE) and the epitope critical in neutralization (ECN). Use of the GP5 ecto-domain and the M protein of PRRSV thus provides a safe and stable delivery of these epitopes of the PRRSV virus to the desired tissues and high antigen expression levels.

The nucleic acids of the present invention may but need not contain the natural sequence of the GP5 ecto-domain of PRRSV strain Olot 91, i.e. SEQ ID NO:1. First of all, one of ordinary skill may find it advantageous to use another strain or isolate of the PRRSV virus which contains a deviating amino acid sequence.

Further, one may find it advantageous to modify the sequence of the GP5 ecto-domain. The sequence may be modified for different reasons. For example, one of ordinary skill could consider modifications of the sequence of the GP5 ecto-domain to further improve stability of the vector. It is generally known that toxic sequences can be removed already by modifying the sequence of 2 or 3 amino acids. In another embodiment, one of ordinary skill may want to amend the sequence of the GP5 ecto-domain to improve the induction of neutralizing antibodies. GP5 protein is the main inducer of neutralizing antibodies. However, previous vaccination approaches based on GP5 expression have shown a delay in the immune response, similar to that obtained after PRRSV infection. It is therefore possible that the GP5 protein stimulates regulatory T cells (Treg) so that these cells generate signals inhibiting the development of the immune response against the GP5 protein. It is for example known that PRRSV infected dendritic cells induce Treg cells (Silva-Campa et al., 2009).

According to one embodiment, the modification of the nucleic acid sequence encoding SEQ ID NO:1 comprises a modification of the immunodominant epitope (IDE), located at positions 30-34 in the GP5 ecto-domain (Ser-Phe-Val-Ala-Gly). This sequence may be modified by substitution or deletion of one or more amino acids. In a preferred embodiment, these five amino acids are deleted.

In a specifically preferred embodiment the nucleic acid of the invention encodes the GP5 ecto-domain and/or M protein of PRRSV, wherein the sequences have been modified to knock out glycosylation sites that interfere with the induction of antibodies. It is known that the glycosylation of GP5 may interfere with the induction of antibodies due to a steric hindrance resulting in a decreased immune response. Therefore, the modified epitopes encoded by the nucleic acids of the invention are able to provide increased stimulatory effects of the immune response compared to naturally occurring epitopes.

In SEQ ID NO:1 the amino acid residues that are glycosylated are positions 46 and 53. Accordingly, the present invention also relates to a modification of the nucleic acid sequence of SEQ ID NO:1, wherein the amino acid at position 46 and 53 has been deleted or substituted. In a preferred embodiment, the nucleic acid comprises the N46S mutation, wherein asparagine at position 46 in the GP5 ecto-domain is replaced by serine. This replacement removes the glycosylation site of the GP5 ecto-domain that interferes most with the induction of antibodies.

In one embodiment, the present invention is therefore directed to a nucleic acid sequence encoding a polypeptide comprising SEQ ID NO:1 that has been modified by deletion of the immunodominant epitope at positions 30-34 and by the N46S substitution. This sequence is shown in SEQ ID NO:12.

In a further alternative embodiment, the modification of the nucleic acid sequence encoding SEQ ID NO:1 comprises a replacement of one or a few amino acids with the amino acid alanine. In a preferred embodiment this replacement reduces the toxicity of the protein. Alternatively, the modification of the nucleic acid sequence encoding SEQ ID NO:1 may comprises an insertion or a deletion of one or a few amino acids.

All modifications of the nucleotide and amino acid sequence are preferably carried out in such a way that the epitopes generating neutralizing anti-PRRSV antibodies are not affected.

In SEQ ID NO: 4 the potential glycosylation sites are the positions 30, 33, 44 and 51. In a preferred embodiment the potential glycosylation sites at position 44 and/or 51 in SEQ ID NO:4 are mutated such that they are not glycosylated by the post-translational glycosylation machinery of the cell.

For similar reasons like for example the reduction of toxicity of the protein, the sequence of the M protein may but need not be the sequence of the M protein of the Olot 91 isolate of PRRSV, i.e. SEQ ID NO:2.

According to a specifically preferred embodiment of the present invention, the nucleic acids comprise more than one copy of SEQ ID NO:1 or a sequence having at least 85% or at least 90% identity to SEQ ID NO:1. Respective nucleic acids thus encode multimers of the GP5 ecto-domain (SEQ ID NO:1) or polypeptides having at least 85% or at least 90% identity with the GP5 ecto-domain (SEQ ID NO:1), wherein these multimers do not comprise an amino acid sequence with 80% or greater homology to amino acids 69 to 201 of the GP5 protein (SEQ ID NO:3). These nucleic acids encode respective multimers of the GP5 ecto-domain and may comprise at least one copy of a nucleic acid which encodes for a polypeptide with 85 or 90% or greater homology to the M protein (SEQ ID NO:2). Proteins generated by these nucleic acids lead to an immunogenic structure providing improved protection because of enhanced neutralizing response. (European PRRSV strain)

According to another embodiment of the present invention, the nucleic acids comprise more than one copy of SEQ ID NO:4 or a sequence having at least 85% or at least 90% identity to SEQ ID NO:4. Respective nucleic acids thus encode multimers of the GP5 ecto-domain (SEQ ID NO:4) or polypeptides having at least 85% or at least 90% identity with the GP5 ecto-domain (SEQ ID NO:4), wherein these multimers do not comprise an amino acid sequence with 80% or greater homology to amino acids 67 to 200 of the GP5 protein (SEQ ID NO:6). These nucleic acids encode respective multimers of the GP5 ecto-domain and may comprise at least one copy of a nucleic acid which encodes for a polypeptide with 85 or 90% or greater homology to the M protein (SEQ ID NO:5). Proteins generated by these nucleic acids lead to an immunogenic structure providing improved protection because of enhanced neutralizing response. (North American PRRSV strain)

According to yet another a preferred embodiment of the present invention, the nucleic acids comprise more than one copy of SEQ ID NO:7 or a sequence having at least 85% or at least 90% identity to SEQ ID NO:7. Respective nucleic acids thus encode multimers of the GP5 ecto-domain (SEQ ID NO:7) or polypeptides having at least 85% or at least 90% identity with the GP5 ecto-domain (SEQ ID NO:7), wherein these multimers do not comprise an amino acid sequence with 80% or greater homology to amino acids 67 to 200 of the GP5 protein (SEQ ID NO:9). These nucleic acids encode respective multimers of the GP5 ecto-domain and may comprise at least one copy of a nucleic acid which encodes for a polypeptide with 85 or 90% or greater homology to the M protein (SEQ ID NO:8). Proteins generated by these nucleic acids lead to an immunogenic structure providing improved protection because of enhanced neutralizing response.

### (Chinese PRRSV strain)

In one aspect these nucleic acids encode two to five repetitions of the sequence encoding the GP5 ecto-domain (SEQ ID NO:1) and one copy of the M protein of PRRSV (SEQ ID NO:2) (European PRRSV strain). In another aspect these nucleic acids encode two to five repetitions of the sequence encoding the GP5 ecto-domain (SEQ ID NO:4) and one copy of the M protein of PRRSV (SEQ ID NO:5) (North American PRRSV strain). In yet another aspect these nucleic acids encode two to five repetitions of the sequence encoding the GP5 ecto-domain (SEQ ID NO:7) and one copy of the M protein of PRRSV (SEQ ID NO:8) (Chinese PRRSV strain). These multimers may be connected by a nucleic acid sequence encoding a flexible linker of two to eight, preferably three to six amino acids. These "linker residues" will improve the flexibility of the epitopes and increase the exposure to the immune system. A specifically preferred linker is the sequence GLY-GLY-PRO-GLY-GLY (SEQ ID NO:10).

In a particularly preferred embodiment of the invention the nucleic acids encode two to five repetitions of the sequence encoding the GP5 ecto-domain (SEQ ID NO:1) and one copy of the M protein of PRRSV (SEQ ID NO:2) (European PRRSV strain).

The nucleic acids of the present invention comprise sequences controlling the expression of the polypeptide coding sequences, such as transcription regulatory sequences. The above sequences (a) and (b) thus may be under the control of the same expression regulatory sequence or the sequences of (a) and (b) may each be present under the control of a different expression regulatory sequence. The sequences regulating expression may comprise transcription regulatory sequences (TRS) and/or internal ribosomal entry site (IRES) sequences to increase transcription and/or translation of the protein coding sequences. Respective TRS and IRES sequences are well known in the art.

The nucleic acids of the present invention may further comprise viral packaging and/or replication sequences. These sequences will generate a viral particle comprising the nucleic acid of the present invention once the nucleic acid has entered a target cell.

In one aspect, the nucleic acids of the present invention encode infectious viral particles. The viral particles obtainable from the association of viral proteins and the nucleic acid sequences are preferably attenuated viral particles.

In a further aspect, the nucleic acids additionally encode the lysosomal targeting signal of lysososmal integral membrane protein-II (LIMPII). According to one embodiment, the nucleic acid may encode a fusion protein consisting of the lysosomal targeting signal of LIMPII, the GP5 ecto-domain and/or the M protein of PRRSV.

The nucleic acids of the present invention may further encode the p35 subunit of Interleukin (IL)-12 or one or more other interleukins. Respective genetic adjuvants may comprise sequences encoding the proteins IL-2, IL-10, IL-12 or granulocyte-macrophage colony-stimulating factor (GM-CSF). The sequences of these polypeptides are well known in the art. The nucleic acids of the present invention may further encode other known sequences promoting mucosal immunity (Plotkin et al., 2005; Toka et al., 2004). In a preferred embodiment the nucleic acids comprise sequences encoding IL-12 or the p35 subunit of IL-12.

In a further embodiment the nucleic acids of the invention may comprise sequences which encode an epitope tag, such as the Hemagglutinin (HA)-tag (SEQ ID NO:11). An "epitope tag" in the sense of the present invention is a short amino acid sequence which is characterized by its high affinity to a corresponding antibody. In a preferred embodiment the epitope tag is expressed under control of the same expression regulatory sequence as the nucleic acid of the invention. In a more preferred embodiment the polypeptide of the present invention is expressed as a fusion protein with the epitope tag. The epitope tag then allows the reliable detection of the tagged protein. The nucleic acid sequence may include a sequence which encodes an epitope tag and a protease cleavage site directly adjacent to the epitope tag such that the epitope tag may be removed after purification.

The nucleic acids of the present invention may be in the form of DNA or RNA.

The present invention is further directed to polypeptides comprising SEQ ID NO:1 or a sequence having at least 85% or at least 90% identity to SEQ ID NO:1, wherein the polypeptide does not comprise the amino acid sequence of SEQ ID NO:3 or a sequence having at least 80% identity to SEQ ID NO:3. The polypeptide may contain other sequences. For example, the polypeptide may contain SEQ ID NO:2 or a sequence having at least 85% or at least 90% identity to SEQ ID NO:2. The polypeptides of the present invention include fusion proteins comprising at least one copy of the amino acid sequence of SEQ ID NO:1 and the amino acid sequence of SEQ ID NO:2, wherein this fusion protein does not comprise the amino acid sequence of SEQ ID NO:3. Again, homologous sequences as defined above are encompassed (European strain).

The present invention is further directed to polypeptides comprising SEQ ID NO:4 or a sequence having at least 85% or at least 90% identity to SEQ ID NO:4, wherein the polypeptide does not comprise the amino acid sequence of SEQ ID NO:6 or a sequence having at least 80% identity to SEQ ID NO:6. The polypeptide may contain other sequences. For example, the polypeptide may contain SEQ ID NO:5 or a sequence having at least 85% or at least 90% identity to SEQ ID NO:5. The polypeptides of the present invention include fusion proteins comprising at least one copy of the amino acid sequence of SEQ ID NO:4 and the amino acid sequence of SEQ ID NO:5, wherein this fusion protein does not comprise the amino acid sequence of SEQ ID NO:6. Again, homologous sequences as defined above are encompassed (North American strain).

The present invention is further directed to polypeptides comprising SEQ ID NO:7 or a sequence having at least 85% or at least 90% identity to SEQ ID NO:7, wherein the polypeptide does not comprise the amino acid sequence of SEQ ID NO:9 or a sequence having at least 80% identity to SEQ ID NO:9. The polypeptide may contain other sequences. For example, the polypeptide may contain SEQ ID NO:8 or a sequence having at least 85% or at least 90% identity to SEQ ID NO:8. The polypeptides of the present invention include fusion proteins comprising at least one copy of the amino acid sequence of SEQ ID NO:7 and the amino acid sequence of SEQ ID NO:8, wherein this fusion protein does not comprise the amino acid sequence of SEQ ID NO:9. Again, homologous sequences as defined above are encompassed (Chinese strain).

In accordance with the present application, the term "fusion protein" is used to refer to a combination of sequences fused at the nucleic acid level in such a manner that a single polypeptide is generated instead of originally at least two separately encoded polypeptide sequences.

In a further embodiment, the present invention relates to vectors comprising the nucleic acids as described above. A "vector" in the sense of the present invention is a nucleic acid that contains an origin of replication such that copies of the nucleic acid can be generated in a host cell expressing a polymerase recognizing the origin of replication.

The vector may be a viral vector, i.e. comprise an origin of replication and/or other sequences derived from a virus. The vector may for example be a replication competent viral vector and comprise all sequences required to replicate the virus in a host cell.

The viral vector is preferably derived from viruses of one of the families *coronaviridae, poxviridae* or *adenoviridae.* The vector may for example be a TGEV vector, such as BAC-TGEV-FL, or a poxvirus vector, for example a vaccinia virus vector. Respective viral vectors are well known in the art.

A replication competent viral vector may be infectious or not. An "infectious" nucleic acid or vector in accordance with the present invention contains all sequences necessary for replication of the nucleic acid and the sequences that generate one or several coat proteins which associate with the nucleic acid to generate a viral particle that will allow infection of other cells.

In an especially preferred aspect, the present invention provides a virus particle that comprises the above nucleic acids and at least one viral coat protein. The virus particle may be live, attenuated or killed and methods to generate attenuated or killed forms of known viruses are well described in the art.

The virus particle may comprise more than one viral coat protein. A corresponding virus particle will be capable of entering a host cell by way of infection. However, the nucleic acid of such a respective virus particle may still be infectious or non-infectious, as it need not encode all of the polypeptides necessary to produce a virus particle. If the nucleic acid is a non-infectious nucleic acid, the virus particle is prepared using a packaging host cell or a helper virus. The use of packaging host cells or helper viruses for obtaining virus particles comprising an incomplete genome of a virus is well known in the art. This way of proceeding has specific advantages, as the virus particle is *per se* infectious (i.e. can infect a cell once) but the nucleic acid is not capable of producing further infectious virus particles. In other words, the sequences derived from the original packaging virus do not encode proteins that will be capable of associating with the nucleic acid to form a new virus particle. These virus particles thus are extremely safe and still provide a high immunogenic response against the epitopes expressed by the nucleic acids.

The present invention further provides host cells comprising a vector of the present invention. The cell may be a bacterial cell, a yeast cell, an insect cell, an animal cell, a mammalian cell or even a human cell. According to a preferred embodiment the cell is a pig cell, particularly a porcine swine testis cell. Respective cells can be obtained as established cell lines and are available for example from ATCC.

The present invention further is directed to pharmaceutical compositions comprising nucleic acids, a vector, a host cell or a viral particle in killed or live form of the present invention as described above. The pharmaceutical composition may further comprise a pharmaceutically acceptable carrier, excipient and/or adjuvants.

In a particularly preferred embodiment the present invention relates to vaccines capable of protecting an animal against the disease caused by an infectious virus comprising a nucleic acid, a vector, a polypeptide, a host cell or a viral particle of the present invention in killed or live form. The vaccine may also comprise pharmaceutically acceptable carriers, excipients and/or adjuvants.

The vaccine may be a modified live vaccine or an inactivated (killed) vaccine.

Modified live vaccines (MLV) are produced from an isolate of viruses or bacteria. Attenuated viruses will not cause disease, but still be capable of replicating in the host cells and thus stimulates immunity (Panley et al., 1989; Pastoret et al., 1997).

Inactivated (killed) vaccines are produced by growing the viruses or viral vectors in bacteria and subsequently isolating and inactivating or killing the organisms using either heat or chemicals. In inactivated (killed) vaccines an adjuvant is added to the antigenic phase of the killed organisms to support stimulation of the immune system, since dead viruses or bacteria are not easily recognized by the immune system in absence of an adjuvant. The adjuvant further holds the killed organisms at the injection site and thus provides sufficient time for the immune cells to respond to it (Panley et al., 1989; Pastoret et al., 1997). Inactivated vaccines may be killed viruses, killed bacteria (also known as bacterins), or killed toxins (or toxoids).

Adjuvants and carriers suitable for administering genetic vaccines and immunogens via the mucosal route are known in the art. Conventional carriers and adjuvants are for example reviewed in Kiyono et al 1996. The addition of chemokines that are used to modulate immune responses are also encompassed by the present invention. Respective compounds and their medical use have been reviewed in Toka et al. 2004. It is specifically advantageous to use one of granulocyte/macrophage colony-stimulating factor (GM-CSF), interleukin-2 (IL-2), IL-12, IL-18. Combinatorial approaches utilizing several cytokines and chemokines might also be applied. In addition, as more is discovered regarding the requirements for memory development of T cells, boosters involving key cytokines such as IL-15 and IL-23 may prove beneficial to long-term maintenance of the memory pool.

The vaccine is preferably suitable for treating a mammal, for example a swine. The vaccination of sows is especially preferred.

In accordance with the present invention vaccines are provided, which are preferably capable of inducing both a systemic immune response and a mucosal immune response against PRRSV.

The vaccine may further be a multivalent vaccine which is capable to provide protection against one or several other pig pathogens. The multivalent vaccine thus may further comprise antigens derived from other viral and/or bacterial pathogens and/or proteins which will provide immunity against pathogens. Examples for antigens from further viral pathogens comprise antigens derived from one of Swine Influenza Viruses, Porcine Parvovirus, Porcine Circovirus Type 2, Classical Swine Fever, African Swine Fever, Foot-and-Mouth Disease, Pseudo-Rabies Virus, Porcine Circovirus Type 1, Porcine Adenoviruses, Porcine Enteroviruses, Porcine Respiratory Coronavirus, Porcine Rotavirus, Encephalomyocarditis Virus, Porcine Epidemic Diarrhea Virus, Blue Eye Disease Viruses, Hepatitis E Virus and/or West Nile Virus, Nipah virus. The use of antigens derived from one or several of Swine Influenza Viruses, Porcine Parvovirus, Porcine Circovirus Type 2, Classical Swine Fever, African Swine Fever and/or Foot-and-Mouth Disease is specifically preferred.

Examples for antigens from bacterial pathogens comprise antigens derived from one of *Mycoplasma hyopneumoniae, Actinobacillus pleuropneumoniae, Actinobacillus* suis, *Haemophilus parasuis, Pasteurella multocida* type A (toxins), *Pasteurella multocida* type D (toxins), *Bordetella bronchiseptica, Isospora suis*, *Brachyspira hyodysenteriae, Brachyspira pilosicoli*, *Lawsonia intracellularis, Erysipelothrix rhusiopathiae, Eschericia coli*, *Salmonella enterica, Mycoplasma hyorinis, Streptococcus suis, Clostridium perfringens, Clostridium difficile, Clostridium novyi, Brucella abortus* and/or *Candidatus helicobacter suis.* The use of antigens derived from one or several of *Mycoplasma hyopneumoniae, Actinobacillus pleuropneumoniae, Actinobacillus* suis, *Haemophilus parasuis, Pasteurella multocida* type A (toxins), *Pasteurella multocida* type D (toxins), *Bordetella bronchiseptica, Isospora suis*, *Brachyspira hyodysenteriae, Brachyspira pilosicoli*, *Lawsonia intracellularis, Erysipelothrix rhusiopathiae, Eschericia coli* and/or *Salmonella enterica* is specifically preferred.

The further viral or bacterial antigen may be present in the multivalent vaccine as an attenuated, killed or inactivated virus or bacterium. Alternatively, the multivalent vaccine may comprise a nucleic acid sequence encoding one or several of the further viral or bacterial antigens. That sequence may be within the same nucleic acid molecule that also encodes the PRRSV sequences or it may be present in the vaccine as a separate nucleic acid molecule.

The multivalent vaccine may further comprise nucleic acid sequences encoding proteins which will confer protection against pig pathogens. Respective nucleic acid sequences could for example code for one or several antibodies having specificity for bacterial or viral diseases. Alternatively or additionally, the nucleic acid may code for the sequence of the porcine prion protein and may thus be used to vaccinate against diseases caused by prions. Again, the sequences encoding proteins which will confer protection against pig pathogens may be within the same nucleic acid molecule that also encodes the PRRSV sequences or may be present in the vaccine as a separate nucleic acid molecule.

These vaccines may be administered in accordance with methods routinely used in the art. Specifically, the vaccines may be administered by intramuscular, intravenous, intranasal, intravaginal, oronasal administration or any other common method known in the art.

The vaccine preferably contains the PRRSV nucleic acids in concentrations producing a live viral titer in the range of about 10⁴ to 10⁹, most preferably about 10⁵ to 10⁸. The live viral titer is determined as plaque forming units (Pfu) in Swine testis (ST) cells.

According to one embodiment of the present invention, the vaccines allow one of ordinary skill to diagnose whether an animal is infected with a wild-type virus or has been vaccinated. According to a further aspect, the present invention is thus directed to methods for diagnosing whether an animal is infected with a virus or has been vaccinated using a vaccine of the present invention, which methods comprise steps, wherein the diagnosis uses antibodies specific for proteins of the wildtype virus not expressed by the vaccine strain. Differentiation of vaccinated animals from infected animals could alternatively be carried out using RT-PCR and sequence markers introduced into the recombinant viral genome.

### BRIEF DESCRIPTION OF THE FIGURES

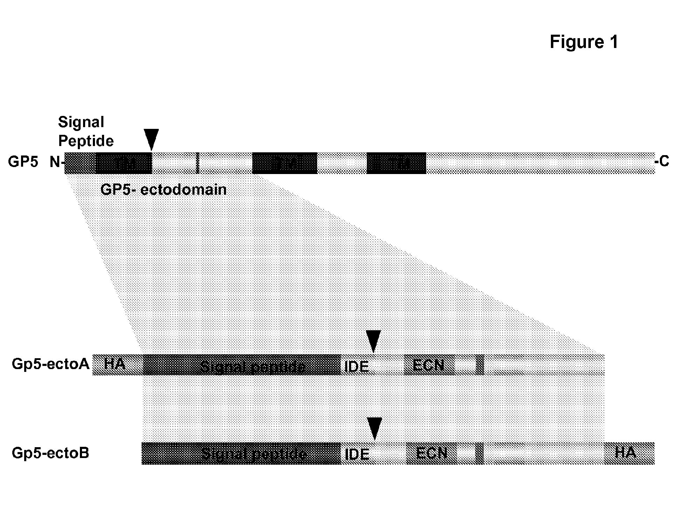

**Figure 1** **shows the structure of GP5.** Schematic representation of PRRSV GP5 protein is shown in the upper part. Antigenic structures, comprising the GP5 ecto-domain, are shown in the bottom part. GP5-ectoA and GP5-ectoB include an HA-tag for detection. Both structures contain the epitopes recognized by non-neutralizing and neutralizing antibodies, named immunodominant epitope (IDE) and epitope critical in neutralization (ECN), respectively. The GP5 fragments also include the two N-glycosylation sites, and the cysteine involved in GP-M heterodimer formation. The signal peptide putative cleavage site is represented by a black arrowhead.

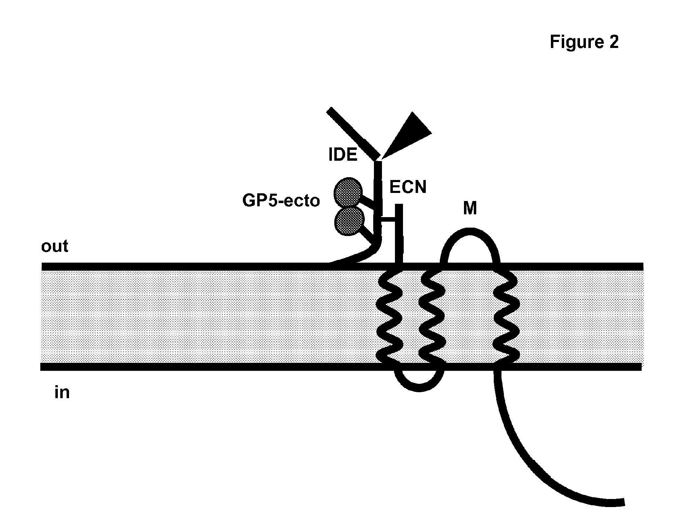

**Figure 2** **shows the predicted topology of a GP5-ecto-M heterodimer in the viral membrane.** PRRSV antigenic structures are comprised by the GP5 protein fragments forming a heterodimer with M protein. The GP5 protein ecto-domain contains the protein motifs relevant in protection, such as the ECN and the IDE. The M protein is relevant for cellular immune response. Disulfide bridges between the GP5-ecto domain and the M protein are shown as black horizontal bars between the two proteins. The figures are not drawn to scale.

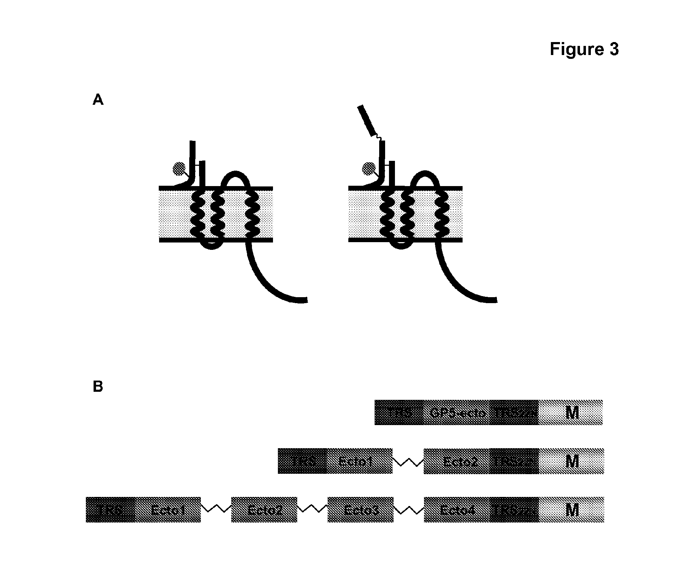

**Figure 3** **shows the multimers of GP5-ecto fragment.** (A) Antigenic structures including M protein and multimers of GP5 ecto-domain (right figure). Further modifications may include selection and improvement of GP5-ecto fragments by removal of glycosylation sites or removal of IDE. (B) Example sequences of multimers of GP5-ecto genes and linker sequences which are under control of the same transcription regulating sequence (TRS). The figures are not drawn to scale.

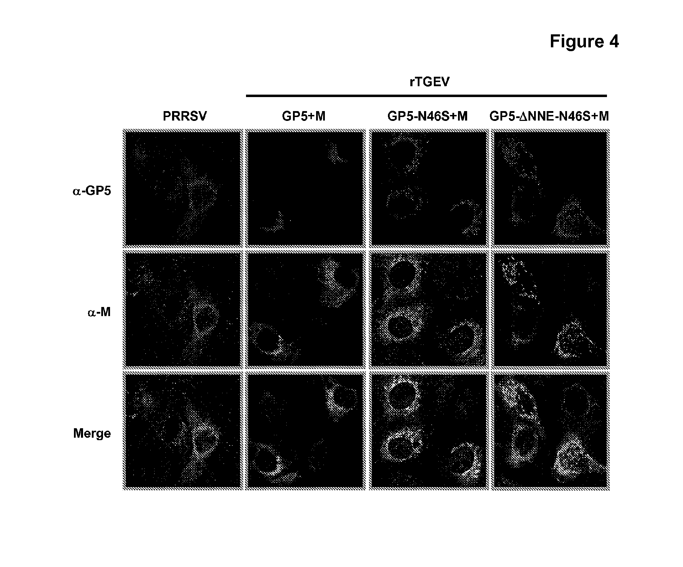

**Figure 4** **shows the Co-localization of GP5 and M proteins.** Expressed PRRSV GP5 and M proteins form heterodimers that have been involved in receptor recognition and viral neutralization. To study if GP5 and M proteins expressed by rTGEVs also colocalize, confocal microscopy analysis was performed. MA-104 or ST cells were infected with PRRSV and the rTGEVs, respectively, and double immunofluorescence staining was performed. Expression of GP5 was detected with a monoclonal antibody specific for GP5 (α-GP5), coupled to a secondary antibody staining red. Expression of M protein was detected with a rabbit antiserum specific for an M protein peptide (α-M), coupled to a secondary antibody staining green. As shown in the merge, colocalization of GP5 and M proteins was observed both in the PRRSV and rTGEV infected cells. Mutant GP5 proteins expressed by rTGEVs also colocalize with M protein.

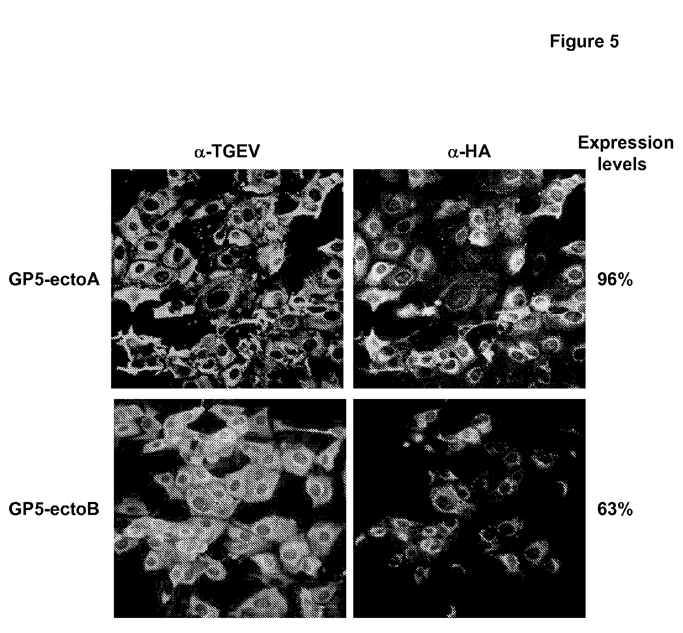

**Figure 5** **shows the expression of HA-tagged GP5-ecto proteins.** ST cells were infected with rTGEVs encoding GP5-ecto-domain with N-terminal HA-tag (GP5-ectoA) or C-terminal HA-tag (GP5-ectoB), and double immunofluorescence staining was performed. TGEV was detected with a rabbit antiserum (α-TGEV), coupled to a secondary antibody staining red. Expression of GP5 protein was detected with a monoclonal antibody specific to the HA peptide (α-HA), coupled to a secondary antibody staining green.

### EXAMPLES

### EXAMPLE 1

### Generation of rTGEV vectors co-expressing GP5-ecto and M proteins of Olot 91 strain of PRRSV.

Antibodies to detect GP5-ecto expression are currently not available, therefore an HA tag (YPYDVPDYA) (SEQ ID NO:11) was introduced amino- or carboxy-terminally to the GP5-ecto domain protein (Figure 1). This tag is small (9 aa) and has been abundantly used to tag and subsequently detect different proteins. The presence of full length GP5-ecto domains has been validated during the following steps using real-time PCR (RT-PCR).

Two different recombinants were obtained, encoding a GP5-ecto fragment with the HA tag at the amino- or carboxy-termini, GP5-ectoA and GP5-ectoB, respectively (Figure 1). Both recombinants contained the protein domains involved in raising an immune response, such as the immunodominant epitope (IDE) and the epitope critical in neutralization (ECN). These GP5-ecto fragments may form a heterodimer with M protein, that - according to results previously obtained with rTGEVs co-expressing full-length GP5 and M proteins (Figure 4) - mimics the PRRSV antigenic structure that is involved in raising an immune response (Figure 2).

### EXAMPLE 2

### Rescue of rTGEV viruses.

Following standard procedures in the lab, rTGEV viruses encoding GP5-ecto and M protein were rescued. Viruses were plaque cloned three times, titrated, and GP5-ecto expression was analyzed by immunofluorescence. Initially recovered clones had viral titers ranging from 10⁴ to 10⁶ pfu/ml, lower than those expected for wild-type rTGEV viruses. GP5-ectoA and GP5-ectoB proteins were expressed in 96% and 63% of the infected cells, as estimated by immunofluorescence (Figure 5).

### EXAMPLE 3

### rTGEV vectors stability analysis.

Viral vectors have to fulfill a strict quality control to be used in subsequent *in vivo* experiments. Among the criteria used to accept a recombinant vector as a vaccine candidate for *in vivo* testing is the level and stability of PRRSV protein expression levels. Further, the recombinant vector has to be efficacious and has to cause no side effects.

rTGEV cloned viruses were passaged eight times in tissue cultures. Viral titers were raised to 10⁸ pfu/ml with serial passages, an expected titer for rTGEV viruses. Swine testis (ST) cells were infected with rTGEVs at a multiplicity of infection (moi) of 1. GP5 ecto-domain protein expression was evaluated by immunofluorescence in passages 4, 8 and 12. In passage 12, GP5-ectoA and GP5-ectoB were expressed in 96% and 63% of the infected cells, respectively (Figure 5). This result indicated that viral titer increased by passaging. Interestingly, GP5 ecto-domain expression was also increased over normal full-length protein expression, suggesting that GP5 stability was improved.

Expression levels of GP5-ectoA were similar to those obtained previously for PRRSV M protein (95%) and GP5-ectoA was stably expressed by the rTGEV vectors. M protein expression levels remained constant after more than 10 virus passages in tissue culture.

### REFERENCES

Botner, A., Strandbygaard, B., Sorensen, K. J., Have, P., Madsen, K. G., Madsen, E. S. & Alexandersen, S. (1997). Appearance of acute PRRS-like symptoms in sow herds after vaccination with a modified live PRRS vaccine. Vet. Rec. 141, 497-499.

Cruz, J.L.G., Zuñiga, S., Becares, M., Sola, I., Ceriani, J.E., Juanola, S., Plana, J. and Enjuanes, L. (2010) Vectored vaccines to protect against PRRSV. Virus Res. 154(1-2): 150-160.

de Haan, C.A., van Genne, L., Stoop, J.N., Volders, H. and Rottier, P.J. (2003) Coronaviruses as vectors: position dependence of foreign gene expression. J. Virol. 77, 11312-11323.

Dokland, T. The structural biology of PRRSV. (2010) Virus Research. Article in Press.

Enjuanes, L., Sola, I., Zuñiga, S. and Ortego, J. (2007) Expression vectors based on coronavirus genomes. In: K.L. Hefferon (Ed), Virus expression vectors, pp. 147-182. Tranworld Research Network, Kerala.

Lopez, O. J. & Osorio, F. A. (2004). Role of neutralizing antibodies in PRRSV protective immunity. Vet Immunol Immunopathol 102, 155-163.

Meng, X. J. (2000). Heterogeneity of porcine reproductive and respiratory syndrome virus: implications for current vaccine efficacy and future vacine development. Vet. Microbiol. 74, 309-329.

Murtaugh, M. P., Xiao, Z. & Zuckermann, F. (1995). Comparison of the structural protein coding sequences of the VR-2332 and Lelystad virus strains of the PRRS virus. Arch. Virol. 140, 1451-1460.

Nielsen, H. S., Oleksiewicz, M. B., Forsberg, R., Stadejek, T., Botner, A. & Storgaard, T. (2001). Reversion of a live porcine reproductive and respiratory syndrome virus vaccine investigated by parallel mutations. J. Gen. Virol. 82, 1263-1272.

Osorio, F. A., Zuckermann, F., Wills, R., Meier, W., Christian, S., Galeota, J. & Doster, A. R. (1998). PRRSV: comparison of commercial vaccines in their ability to induce protection against current PRRSV strains of high virulence. In Allen D. Lennan Swine Conf., pp. 179-182.

Panley R., Hoglund S., Prasad G. (Eds.) (1989). Progress in Vaccinology. Veterinary Vaccines Volume 4, Springer Verlag.

Pastoret P.P., Blancou P., Vannier, Verschueren C. (Eds.) (1997) Veterinary Vaccinology, Elsevier Science B.V.

Plotkin, S.A. (2005) Vaccines: past, present and future. Nat. Med. 11, S5-S11.

Shi, M., Lam, T.T.-Y., Hon, C.-C., Hui, R.K.-H., Faaberg, K.S., Wennblom, T., Murtaugh, M.P., Stadejek, T., Leung, F.C.-C. Molecular epidemiology of PRRSV: A phylogenetic perspective. (2010) Virus Research.

Silva-Campa, E., Flores-Mendoza, L., Resendiz, M., Pinelli-Saavedra, A., Mata-Haro, V., Mwangi, W. and Hernandez, J. (2009) Induction of T helper 3 regulatory cells by dendritic cells infected with porcine reproductive and respiratory syndrome virus. Virology 387, 373-379.
Toka, F.N., D., P.C. and T., R.B. (2004) Molecular adjuvants for mucosal immunity. Immunol Rev. 199, 100-112.

Wensvoort, G., Terpstra, C, Pol, J. M., ter Laak, E. A., Bloemraad, M., de Kluyver, E. P., Kragten, C, van Buiten, L., den Besten, A., Wagenaar, F. & et al. (1991). Mystery swine disease in The Netherlands: the isolation of Lelystad virus. Vet. Q. 13, 121-130.

## Claims

1. Nucleic acid comprising:
(a) a sequence encoding a polypeptide comprising the amino acid sequence of SEQ ID NO:1 or a sequence having at least 85% identity to SEQ ID NO:1, which sequence includes at least one amino acid capable of forming a disulfide bridge; and
(b) a sequence encoding a polypeptide comprising the amino acid sequence SEQ ID NO:2 or a sequence having at least 85% identity to SEQ ID NO: 2;
(c) wherein the nucleic acid does not comprise a sequence encoding a polypeptide comprising the amino acid sequence of SEQ ID NO:3 or a sequence having at least 80% identity to SEQ ID NO:3.

2. Nucleic acid comprising:
(a) a sequence encoding a polypeptide comprising the amino acid sequence of SEQ ID NO:4 or a sequence having at least 85% identity to SEQ ID NO:4, which sequence includes at least one amino acid capable of forming a disulfide bridge; and
(b) a sequence encoding a polypeptide comprising the amino acid sequence SEQ ID NO:5 or a sequence having at least 85% identity to SEQ ID NO:5;
(c) wherein the nucleic acid does not comprise a sequence encoding a polypeptide comprising the amino acid sequence of SEQ ID NO:6 or a sequence having at least 80% identity to SEQ ID NO:6.

3. Nucleic acid comprising:
(a) a sequence encoding a polypeptide comprising the amino acid sequence of SEQ ID NO:7 or a sequence having at least 85% identity to SEQ ID NO:7, which sequence includes at least one amino acid capable of forming a disulfide bridge; and
(b) a sequence encoding a polypeptide comprising the amino acid sequence SEQ ID NO:8 or a sequence having at least 85% identity to SEQ ID NO:8;
(c) wherein the nucleic acid does not comprise a sequence encoding a polypeptide comprising the amino acid sequence of SEQ ID NO:9 or a sequence having at least 80% identity to SEQ ID NO:9.

4. Nucleic acid according to any of claims 1-3, wherein said nucleic acid encodes a fusion protein comprising the sequences of (a) and (b).

5. Nucleic acid according to claim 1 or 4, wherein the nucleic acid comprises more than one copy of SEQ ID NO:1 or a sequence having at least 85% identity to SEQ ID NO:1.

6. Nucleic acid according to claim 2 or 4, wherein the nucleic acid comprises more than one copy of SEQ ID NO:4 or a sequence having at least 85% identity to SEQ ID NO:4.

7. Nucleic acid according to claim 3 or 4, wherein the nucleic acid comprises more than one copy of SEQ ID NO:7 or a sequence having at least 85% identity to SEQ ID NO:7.

8. Nucleic acid according to claim one of claims 1 to 7, wherein the sequences of (a) and (b) are under the control of the same expression regulatory sequence or the sequences of (a) and (b) are each under the control of a different expression regulatory sequence.

9. Nucleic acid according to any of claims 1 to 8, wherein the sequence encoding said neutralizing epitope has been modified to knock out glycosylation sites that interfere with induction of antibodies.

10. Nucleic acid according to any of claims 1 to 9, wherein the nucleic acid further comprises one or more viral packaging and/or replication sequences.

11. Nucleic acid according to claim 10, wherein the infectious viral particles obtainable from the association of viral proteins and the nucleic acid sequences are attenuated viral particles.

12. Nucleic acid according to any one of claims 1 to 11, wherein the nucleic acid further encodes a lysosomal targeting signal of lysosomal integral membrane protein-II (LIMPII).

13. Nucleic acid according to claim 12, wherein said nucleic acid encodes a fusion protein consisting of the lysosomal targeting signal of LIMPII and GP5-ecto and/or M protein of PRRSV.

14. Nucleic acid according to any one of claims 1 to 13, wherein the nucleic acid further encodes the p35 subunit of IL-12 or one or more other interleukins.

15. Vector comprising a nucleic acid according to one of claims 1 to 14.

16. Vector according to claim 15, wherein the vector is a cDNA vector.

17. Vector according to claim 15 or 16, wherein the vector is a poxvirus vector, for example a vaccinia virus vector, or coronavirus vector, for example a BAC-TGEV vector.

18. Host cell comprising a vector according to one of claims 15 to 17, wherein the cell is a bacterial cell, a yeast cell, an insect cell, an animal cell, a mammalian cell or a human cell.

19. Virus particle comprising a nucleic acid according to any of claims 1 to 13 and at least one viral coat protein.

20. Virus particle according to claim 19, wherein the virus particle is a killed virus particle.

21. Polypeptide comprising the amino acid sequence of SEQ ID NO:1 or a sequence having at least 85% identity to SEQ ID NO:1, wherein the polypeptide does not comprise SEQ ID NO:3 or a sequence having at least 80% identity to SEQ ID NO:3.

22. Polypeptide comprising the amino acid sequence of SEQ ID NO:4 or a sequence having at least 85% identity to SEQ ID NO:4, wherein the polypeptide does not comprise SEQ ID NO:6 or a sequence having at least 80% identity to SEQ ID NO:6.

23. Polypeptide comprising the amino acid sequence of SEQ ID NO:7 or a sequence having at least 85% identity to SEQ ID NO:7, wherein the polypeptide does not comprise SEQ ID NO:9 or a sequence having at least 80% identity to SEQ ID NO:9.

24. Pharmaceutical preparation comprising
(a) a nucleic acid according to one of claims 1 to 14, a vector according to one of claims 15 to 17, a host cell according to claim 18, a live, attenuated or killed virus particle according to claim 19 to 20 or a polypeptide according to claim 21 to 23, and
(b) a pharmaceutically acceptable carrier, excipient and/or adjuvants.

25. Vaccine capable of protecting an animal against PRRSV comprising
(a) a nucleic acid according to one of claims 1 to 14, a vector according to one of claims 15 to 17, a host cell according to claim 18, a live, attenuated or killed virus particle according to claim 19 to 20 or a polypeptide according to claim 21 to 23; and
(b) a pharmaceutically acceptable carrier, excipient and/or adjuvants.

26. Vaccine according to claim 25, wherein the vaccine is a multivalent vaccine capable to provide protection against one or several pig pathogens other than PRRSV.

27. Vaccine according to one of claims 25 to 26, wherein the vaccine is capable of inducing both a systemic immune response and a mucosal immune response against infectious viral agents.
